# EUROPEAN PATENT SPECIFICATION

(11) **EP 2 936 218 B1**
(45) Date of publication and mention of the grant of the patent: **22.02.2017**
(21) Application number: 13830100.7
(22) Date of filing: 16.12.2013
(51) Int. Cl.: G02B 1/04

(54) **PROTECTIVE COMPOSITION**
SCHUTZZUSAMMENSETZUNG
COMPOSITION PROTECTRICE

(30) Priority: 20.12.2012 US 201261739780 P
(43) Date of publication of application: 28.10.2015
(73) Proprietor: Philips Lighting Holding B.V., 5656 AE Eindhoven (NL)
(72) Inventor: HIKMET, Rifat Ata Mustafa, NL-5656 AE Eindhoven (NL); VAN BOMMEL, Ties, NL-5656 AE Eindhoven (NL)
(74) Representative: van Eeuwijk, Alexander Henricus Waltherus
(86) International application number: PCT/IB2013/060991
(87) International publication number: WO 2014/097120

(56) References cited:
- WO-A1-2011/065044

## Description

### FIELD OF THE INVENTION

The present invention relates to materials and components for use in optoelectronic devices for protection against detrimental substances, and to light emitting arrangements comprising such materials and components.

### BACKGROUND OF THE INVENTION

The presence or formation of corrosive gas, such as H₂S, is a problem in many instances. For example, in lighting arrangements using silver reflectors, the reaction of silver with sulfide gas present in the surrounding atmosphere leads to darkening of the silver and hence impaired function of the reflector.

In some light emitting arrangements based on phosphor-converted light sources, sulfide gas may be released from sulfur-containing phosphor materials, typically phosphors based on sulfide materials, such as calcium sulfide (CaS), strontium sulfide (SrS), zinc sulfide (ZnS) or combinations thereof such as (Ca,Sr)S. Sulfide based phosphor are particularly sensitive to water and may react to form calcium or strontium hydroxide and sulfide gas. In addition to the darkening of silver reflectors, the release of H₂S is also a problem because it is a toxic, flammable and foul-smelling gas. Furthermore, phosphors based on Ca or Sr selenide or sulfide may upon reaction with water also release small amounts of selenide gas, which is highly toxic.

It would be desirable not only to prevent corrosive and/or toxic gas from reaching a silver layer, but also from escaping from a device.

In order to prevent silver reflectors from darkening, protective layers have been proposed. However, such protective layers usually have poor scratch resistance, and the application of an insulating protective layer directly on a silver layer may also be undesirable since silver is often used as an electrical conductor and must be wired. Furthermore, making such layers without pinholes, through which gas may leak, is almost impossible.

US 2009/0121180 suggests a protective composition comprising a silica-loaded silicone composition which can be used for encapsulating an optoelectronic device. The composition comprises siloxane-grafted silica particles and yields a cured silicone composition that is highly transparent and has low permeability to corrosive gases. However, low permeability does not solve the problem but only delays it.

Hence, there is a need in the art for improvements regarding the prevention or reduction of detrimental effects of toxic and/or corrosive gases in lighting arrangements.

### SUMMARY OF THE INVENTION

It is an object of the present invention to overcome this problem, and to provide means to capture sulfur, suitable for use in an optoelectronic device.

According to a first aspect of the invention, this and other objects are achieved by a protective composition for use in an optoelectronic device, which protective composition comprises an organometallic getter capable of reacting with sulfur and/or selenium to form crystals, typically nanocrystals, containing a metal and sulfur and/or selenium. The crystals are formed according to the following equation:

H₂Z + MX → MZ + XH₂

wherein H₂Z represents sulfide gas or selenide gas, M represents a metal moiety of the organometallic getter selected from the group consisting of copper, iron, cadmium, lead and zinc, X represents an organic moiety of the organometallic getter, and MZ represents the formed crystals.

Generally, the term "getter" denotes a reactive substance that interacts with a target substance to immobilize said target substance, by adsorption and/or chemical binding. The getter used in the present invention primarily acts by chemically binding the target substance. In the present invention, the target substance is typically sulfur/sulfide or selenium/selenide. The chemical interaction between the getter and the target substance may be, typically, ionic binding.

The removal of sulfide gas by reaction with a getter is advantageous because the reaction results in the formation of stable crystals, and does not merely reduce the permeation rate of the gas. The resulting crystals typically have high transparency and low light absorption. In embodiments of the invention, the composition comprising the getter (before reaction with sulfide gas) may also have low light absorption, which makes the composition including the getter particularly suitable for use in optoelectronic devices. In addition, the composition is easy to produce using conventional technology.

In embodiments of the invention the organometallic getter may comprise a metal selected from the group consisting of copper, iron, cadmium, lead, and zinc. These metals are capable of forming suitable crystal complexes with sulfur and/or selenium. In particular, the organometallic getter may comprise zinc. Zinc sulfide or zinc sulfide crystals have high transparency. Advantageously, zinc is a nontoxic material.

In embodiments of the invention the organometallic getter may comprise an organic anion. The organic anion is typically complexed to the metal, typically a metal ion (e.g. zinc ion).

In embodiments of the invention, the protective composition further comprises a matrix comprising a polymer. The matrix acts as a carrier for the organometallic getter. The organometallic getter may be chemically attached to the polymer, for example by means of the organic moiety (organic anion) which may be attached to the polymer as a side chain. For example, the polymer may comprise silicone or a silicone derivative, or an acrylate based polymer.

In embodiments of the invention, the protective composition may further comprise a wavelength converting material. Hence, the composition may combine protection against sulfide or selenide gas with wavelength converting properties. Such embodiments may be particularly useful when the wavelength converting material itself comprises sulfur or selenium.

In embodiments of the invention, the protective composition may be in the form of a layer.

Hence, in another aspect, the invention provides a protective layer comprising the protective composition described herein.

In a further aspect, the invention provides a layer assembly comprising a protective composition or a protective layer as described herein and a wavelength converting layer comprising a wavelength converting material. The wavelength converting material, provided as a separate layer or contained within the protective composition or the protective layer, may comprise sulfur and/or selenium.

In another aspect, the invention provides an optical component comprising a protective composition as described herein. The optical component may thus combine optical functionality with protection against sulfide or selenide gas.

In yet another aspect, the invention provides a light emitting arrangement comprising at least one solid state light source and a protective composition, a protective layer, or a layer assembly as described herein. By including the protective composition in a light emitting arrangement, sulfide gas can effectively be prevented both from damaging sensitive structures within the arrangement, as well as from leaking out to the exterior.

In embodiments of the invention, the light emitting arrangement further comprises a silver layer at least partly covered by the protective composition or protective layer. The silver layer may be a silver reflector. Furthermore, in embodiments of the invention, a wavelength converting material may be arranged to receive light emitted by said solid state light source, wherein said wavelength converting material is at least partly covered by said protective layer. In some embodiments, the protective composition may be arranged between a silver layer and a wavelength converting layer.

Lastly, the invention provides the use of an organometallic getter for capturing corrosive or toxic gas, in particular sulfide or selenide gas, released from a wavelength converting material.

It is noted that the invention relates to all possible combinations of features recited in the claims.

### BRIEF DESCRIPTION OF THE DRAWINGS

This and other aspects of the present invention will now be described in more detail, with reference to the appended drawings showing embodiment(s) of the invention.
Figure 1 illustrates the reaction scheme between H₂S gas and an organometallic getter bound to a polymer matrix.
Figure 2 is a cross-sectional side view of a light emitting arrangement according to embodiments of the invention, comprising a thin layer of a protective composition.
Figure 3 is a cross-sectional side view of a light emitting arrangement according to embodiments of the invention, comprising a protective composition which may function as an encapsulant.
Figure 4 is a schematic side view of a light emitting arrangement according to embodiments of the invention, comprising a light source, a wavelength converting layer and a protective layer.
Figure 5 is a cross-sectional side view of a light emitting arrangement according to embodiments of the invention, comprising a plurality of light sources arranged in a reflective chamber, and a protective layer.
Figure 6 shows a side view of a bulb replacement lamp that may comprise a light emitting arrangement according to embodiments of the invention.
Figure 7 shows a perspective view of a luminaire that may comprise a light emitting arrangement according to embodiments of the invention.

As illustrated in the figures, the sizes of layers and regions are exaggerated for illustrative purposes and, thus, are provided to illustrate the general structures of embodiments of the present invention. Like reference numerals refer to like elements throughout.

### DETAILED DESCRIPTION

The present invention will now be described more fully hereinafter with reference to the accompanying drawings, in which currently preferred embodiments of the invention are shown. This invention may, however, be embodied in many different forms and should not be construed as limited to the embodiments set forth herein; rather, these embodiments are provided for thoroughness and completeness, and fully convey the scope of the invention to the skilled person.

The present invention is based on the realization that an organometallic getter capable of forming a crystal complex with sulfur can be used for capturing H₂S gas. Thereby adverse effects of this gas can be reduced, in particular in light emitting arrangements. Advantageously the organometallic getter can also form a crystal complex with selenium and thereby also capture H₂Se gas. Several metals can form crystal complexes with sulfur and/or selenium, even nanocrystals, that have no or only low light absorption.

Examples of suitable metals of the organometallic getter include copper, iron, cadmium, lead and zinc. Zinc may be particularly advantageous since zinc sulfide nanoparticles have almost no light absorption and also because zinc is non-toxic.

The organic moiety of the organometallic getter is an organic anion, typically the anion of an organic salt. Examples of suitable anions include acetonate anions, carboxylate anions, phthalate anions, and sulfonate anions that may form metal complexes (typically salts). Examples of such salts include zinc acetate, cadmium acetate, zinc benzoate, lead naphtalate, iron(II)acetate, ferric acetonate and zinc trifluoromethane sulfonate.

In general, the reaction between an organometallic getter including a metal ion and sulfide gas may be as follows (Equation I):

H₂S + MX → MS + XH₂ Equation 1

in which M represents the metal atom of the organometallic getter and X represents the organic anions of the organometallic getter. Depending on the oxidation states of the metal and the organic anion Formula I may have to be adapted with respect to the relative amounts of M, X and H₂S. The corresponding equation for the reaction between the organometallic getter and selenide gas may be as follows (Equation II):

H₂Se + MX → MSe + XH₂ Equation II

in which M represents the metal atom of the organometallic getter and X represents the organic anions of the organometallic getter. Depending on the oxidation states of the metal and the organic anion Formula I may have to be adapted with respect to the relative amounts of M, X and H₂Se.

As an example, the reaction between an organometallic getter comprising zinc(II) and H₂S may be as follows (Equation Ia):

H₂S + ZnX → ZnS + XH₂ Equation Ia

in which X represents the organic anions of the organometallic getter. The corresponding reaction between zinc and selenide gas may be as follows (Equation IIa):

H₂Se + ZnX → ZnSe + XH₂ Equation IIa

in which X represents the organic anions of the organometallic getter.

For instance, the reaction between zinc acetate as the organometallic getter and H₂S is as follows (Equation Ib):

H₂S + Zn(CH₃CO₂)₂ → ZnS+(CH₃CHO₂)₂ Equation Ib.

The metal sulfide or metal selenide reaction product formed by reaction of the organometallic getter with sulfide gas or selenide gas, respectively, may be present in the form of crystals, for example nanocrystals. Advantageously, such crystals, in particular nanocrystals, may have high transparency and may have low or no light absorption. Nanocrystals are particularly advantageous as the absorption band shifts to lower wavelengths with decreasing particle size.

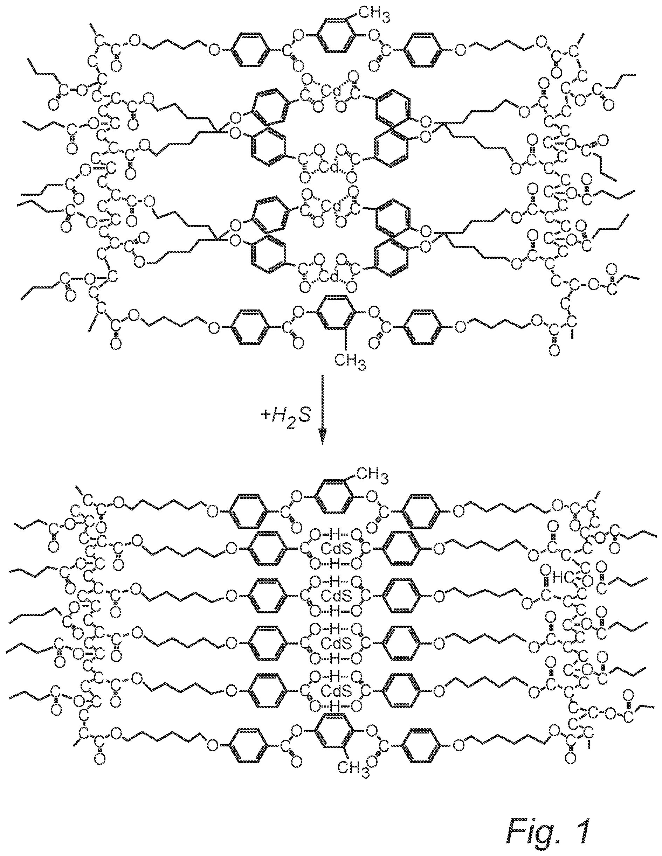

According to embodiments of the invention, the reaction between an organometallic getter comprising cadmium and H₂S gas may be as outlined in Fig. 1. Before reaction with H₂S, the organometallic getter comprises cadmium benzoate bound to an acrylate polymer network. Upon reaction with H₂S, cadmium sulfide (CdS) nanocrystals are formed, surrounded by the polymer matrix containing benzoic acid linked to the acrylate polymer network.

The organometallic getter is typically incorporated in a carrier matrix, which may be a polymer matrix or a siloxane-based matrix. Suitably the matrix material may be capable of forming a complex with the metal ion of the organometallic getter. Examples of matrix materials contemplated for use in embodiments of the present invention include polysiloxane-based materials, such as poly(dimethyl siloxane) and derivatives thereof. A polysiloxane-based material may be crosslinked. For instance, the following material are capable of forming complexes with zinc and are therefore advantageously used in combination with an organometallic getter comprising zinc: carboxydecyldimethyl terminated poly(dimethyl siloxane), carboxypropyl terminated poly(dimethyl siloxane), trimethyl silylpropionic acid, 1,3-bis(3-carboxypropyl)tetramethyldisiloxane, and 2-(trimethyl silyl)acetic acic.

Typically, the matrix material may be a material that can be formed in to a layer, optionally a self-supporting sheet. A composition of matrix material, including the organometallic getter, can be applied onto a surface, such as the surface of an LED or the surface of a wavelength converting element or layer, to form a coating layer using conventional surface coating techniques. Alternatively, the composition can be extruded, injection molded or compression molded. The composition may form a functional optical component such as a dome, a lens or a collimator optionally in direct contact with an LED. Optionally the composition typically in the form of a layer may be combined with one or more other functional layers such as a wavelength converting layer or a reflective layer, thus forming a layer assembly. For example, the composition comprising the organometallic getter and the matrix may be applied, e.g. by screen printing, or other conventional coating technique on top of a wavelength converting layer or a reflective layer. In the case of packaged LEDs, the matrix carrying the organometallic getter can be brought in liquid form (typically, not completely polymerized) into the cup containing the LEDs, followed by curing of the matrix by polymerization and/or cross-linking.

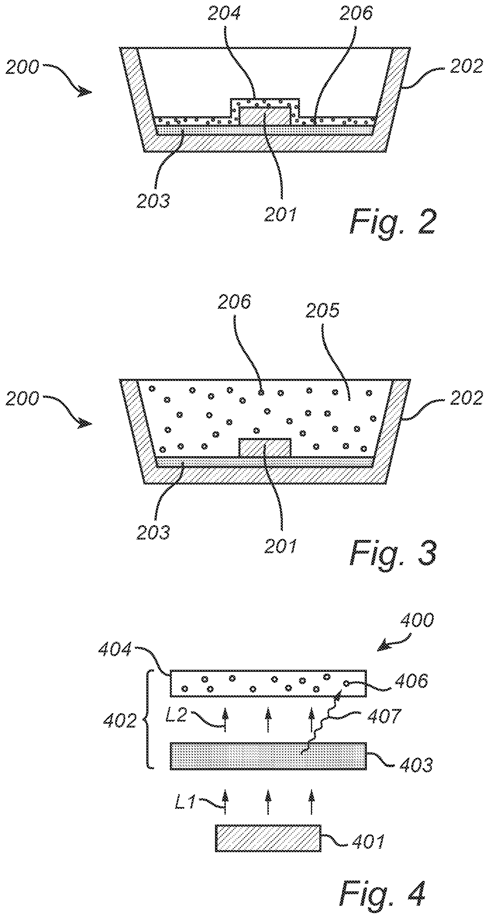

The organometallic getter, optionally incorporated in a carrier matrix, may be provided as a protective layer covering or encapsulating at least a part of or a component of a light emitting arrangement. Figure 2 schematically illustrates a light emitting arrangement comprising a protective layer according to an embodiment of the invention. In Figure 2, which is a cross-sectional side view, is shown a light emitting diode (LED) package 200 comprising an LED 201 arranged in a reflective cup 202. The bottom of the reflective cup, on which the LED is mounted, has a reflective silver layer 203. The reflective silver layer 203 and the LED 201 are covered with a thin protective layer 204 of substantially uniform thickness, comprising an organometallic getter typically distributed in a carrier matrix as described above. The protective layer may be transparent or translucent. The organometallic getter of the protective layer 204 is capable of capturing H₂S gas and/or H₂Se gas released from the wavelength converting layer or otherwise present within the light emitting arrangement.

Figure 3 shows another embodiment of a light emitting arrangement, similar to the embodiment shown in Figure 2 but instead of the thin protective layer 204 having a thicker layer 205 of the protective composition filling the interior of the reflective cup 202, thus functioning as an encapsulant.

It is contemplated that in a so-called chip-on-board package, a plurality of LED arranged on a printed circuit board mounted on the reflective layer (instead of the single LED 201 shown in Figures 1 and 2) may be covered with a protective layer of suitable thickness, uniform or non-uniform.

A composition comprising the organometallic getter, typically incorporated in a matrix, may further comprise at least one wavelength converting material, commonly known as a phosphor. Such a composition may thus form a protective layer which also has wavelength converting properties. Additionally or alternatively, a composition comprising the organometallic getter may comprise scattering element, for example scattering particles such as particles of Al₂O₃ or TiO₂.

In general, the wavelength converting material used in embodiments of the present invention may be an inorganic wavelength converting material or an organic wavelength converting material. Examples of inorganic wavelength converting materials may include, but are not limited to, cerium (Ce) doped yttrium aluminum garnet (Y₃Al₅O₁₂:Ce³⁺, also referred to as YAG:Ce or Ce doped YAG) or lutetium aluminum garnet (LuAG, Lu₃Al₅O₁₂), α-SiAlON:Eu²⁺ (yellow), and M₂Si₅N₈:Eu²⁺ (red) wherein M is at least one element selected from calcium Ca, Sr and Ba. Furthermore, a part of the aluminum of YAG:Ce may be substituted with gadolinium (Gd) or gallium (Ga), wherein more Gd results in a red shift of the yellow emission. Other suitable wavelength converting materials may include (Sr_{1-x-y}BaₓCa_{y})_{2-z}Si₅₋ₐAlₐN₈₋ₐOa:Eu²⁺ wherein 0 ≤a<5, 0≤x≤1, 0≤y≤ 1 and 0<z≤ 1, and (x+y)≤1, such as Sr₂Si₅N₈:Eu²⁺ which emits light in the red range.

Examples of inorganic wavelength converting materials include quantum dots or quantum rods. Quantum dots are small crystals of semiconducting material generally having a width or diameter of only a few nanometers. When excited by incident light, a quantum dot emits light of a color determined by the size and material of the crystal. Light of a particular color can therefore be produced by adapting the size of the dots. Most known quantum dots with emission in the visible range are based on cadmium selenide (CdSe) with a shell such as cadmium sulfide (CdS) and zinc sulfide (ZnS). Cadmium free quantum dots such as indium phosphode (InP), and copper indium sulfide (CuInS2) and/or silver indium sulfide (AgInS2) can also be used. Quantum dots show very narrow emission band and thus they show saturated colors. Furthermore the emission color can easily be tuned by adapting the size of the quantum dots.

Any type of quantum dot known in the art may be used in the present invention. However, it may be preferred for reasons of environmental safety and concern to use cadmium-free quantum dots or at least quantum dots having a very low cadmium content.

Examples of suitable organic phosphor materials are organic luminescent materials based on perylene derivatives, for example compounds sold under the name Lumogen^{®} by BASF. Examples of suitable compounds include, but are not limited to, Lumogen^{®} Red F305, Lumogen^{®} Orange F240, Lumogen^{®} Yellow F083, and Lumogen^{®} F170.

In embodiments of the invention, narrow band emitting materials, including sulfide based wavelength converting materials, optionally europium doped, may be used to great advantage.

In fact, incorporation of a wavelength converting material into a composition comprising the organometallic getter may be particularly advantageous when the wavelength converting material contains sulfur and/or selenide and hence may give rise to sulfide and/or selenide gas. Examples of such wavelength converting materials include phosphors based on, for example, calcium sulfide, strontium sulfide, calcium strontium sulfide, calcium selenide sulfide, and calcium strontium selenide sulfide.

Another embodiment of the invention is schematically depicted in Figure 4. This figure shows a side view of the general layer structure of a light emitting arrangement 400 including a solid state light source 401 and a layer assembly 402 comprising a wavelength converting layer 403 and a protective layer 404. The light source 401 is adapted to emit primary light in the direction of the wavelength converting layer 403. The wavelength converting layer 403 receives light emitted by the light source 401 and converts this light into secondary, converted light.

The protective layer 404 comprises an organometallic getter 406, typically distributed in a matrix as described above. The wavelength converting layer, which may contain a wavelength converting material comprising sulfur and/or selenium, may be arranged between the light source 401 and the protective layer 404. Hence, the protective layer is arranged on the light output side of the wavelength converting layer, i.e. on the side intended to face a viewer of the light emitting arrangement 400. Optionally, the wavelength converting layer may be partly or fully covered, or even embedded, by the protective composition.

The organometallic getter of the protective layer 404 is capable of capturing H₂S gas 407 and optionally H₂Se gas released from the wavelength converting layer or otherwise present adjacent the protective layer.

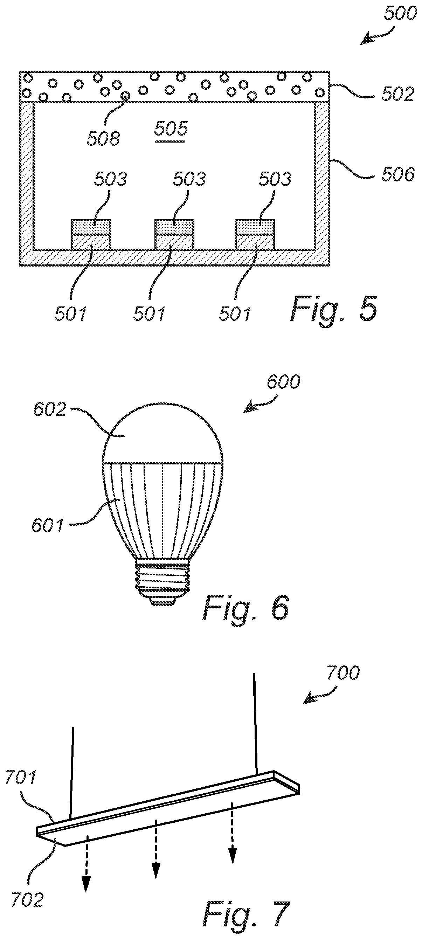

Although not shown in Figure 4, a light emitting arrangement may include a reflective cup as shown in Figures 2 and 3, and may be sealed, optionally by the protective layer 404. In embodiments of the invention, schematically illustrated in Figure 5, a light emitting arrangement 500 may comprise a plurality of solid state light sources 501 arranged within a reflective chamber 505, also referred to as a light mixing chamber, defined by at least one reflective wall 506. A protective layer 502 comprising an organometallic getter 508 as described above forms part of a light exit window 507 of the light mixing chamber 505. In the embodiment of Figure 5, a wavelength converting layer 503 is provided directly on top of each light source 501, but alternatively or additionally a wavelength converting material may be included within the protective layer, or arranged as a wavelength converting layer adjacent the protective layer 502. Indeed, in embodiments of the invention, the layer assembly 402, the wavelength converting layer 403, 503 and/or the protective layer 404, 502 may form part of the light exit window through which light may exit a light mixing chamber.

Referring again to Figure 4, the layer assembly 402 comprises at least a protective layer as described above. Typically it also comprises at least one additional layer, which may be a wavelength converting layer 403. Alternatively, in embodiments where the protective layer also contains wavelength converting material as described above, the additional layer may be another optically functional layer, such as a scattering layer. In some embodiments of the invention, the at least one additional layer may comprise a support layer, e.g. comprising matrix material without any organometallic getter. The at least one additional layer may be positioned on any suitable side of the protective layer 404.

In embodiments of the invention, the layer assembly may comprise the protective layer and the at least one additional layer arranged in direct contact with each other, thus forming a multilayer structure. In other embodiments however the layer assembly may comprise at least two layers mutually spaced apart. For example, as shown in Figure 4, the protective layer 404 and the wavelength converting layer 403 may be arranged at a distance from each other, separated by a gap. Alternatively, in embodiments comprising more than two layers, for example a protective layer, a wavelength converting layer and a further layer, any two of these layers may be arranged in direct contact with each other while the third layer may be spaced apart from the two layers.

Furthermore, in embodiments of the invention, a layer assembly comprising a protective layer as described above and at least one additional layer may be arranged directly on top of a light source, such as an LED. Alternatively one or more layers of the layer assembly, and optionally the whole layer assembly, may be arranged at a distance from the light source.

Similarly, embodiments of the invention where the protective layer comprises a wavelength converting material in addition to the organometallic getter, the protective layer may be arranged directly on a light source, or at a distance from a light source, typically an LED.

A light emitting arrangement according to embodiments of the invention may typically comprise a solid state light source. The solid state light source may be a light emitting diode (LED) or a laser diode. Alternatively the light source may be an organic light emitting diode (OLED). In some embodiments the solid state light source may be a blue light emitting LED, such as GaN or InGaN based LED, for example emitting primary light of the wavelength range from 440 to 460 nm. Alternatively, the solid state light source may emit UV or violet light. Blue, violet or UV LEDs can be combined with one or more wavelength converting material such that light source light is converted into light of longer wavelength(s) by the wavelength converting material(s).

The protective layer and the light emitting arrangement according to embodiments of the invention described herein may be employed in a variety of lighting applications and devices. For example a light emitting arrangement according to embodiments of the invention may be used in an LED based bulb replacement lamp, for replacement of conventional light incandescent bulbs. A bulb replacement lamp typically comprises a light emitting arrangement, which may be a light emitting arrangement as described herein. Figure 6 shows a side view of a bulb replacement lamp 600 comprising a base member 601 adapted to allow electrical connection to the light source, including a fitting for a conventional socket. On the top surface of the base member a light emitting arrangement is mounted (not shown). Typically, the light emitting arrangement may comprise a plurality of light sources, usually LEDs. A transparent or translucent cover 602, optionally shaped like a conventional bulb, may be attached to the base member 601 to cover the light emitting arrangement. A protective layer or the layer assembly according to embodiments of the invention may be arranged at any suitable position between the light source(s) and the cover 602. It is contemplated that the protective layer or layer assembly according to embodiments of the invention may be arranged on an inner surface of the transparent or translucent cover 602.

Furthermore, the protective layer or the light emitting arrangement according to embodiments of the invention may be employed in a luminescent tube replacement lamp, also referred to as a TL replacement lamp or tube. A TL replacement lamp usually comprises an elongated, e.g. tubular, at least partially transparent or translucent enclosure enclosing a light emitting arrangement which may be a light emitting arrangement as described herein. The elongated enclosure is typically provided with electrical contacts allowing electrical connection to the light source(s) Typically, the light emitting arrangement may comprise a plurality of light sources, usually LEDs. A protective layer or the layer assembly according to embodiments of the invention may be arranged at any suitable position between the light source(s) and the enclosure. It is contemplated that the protective layer or layer assembly according to embodiments of the invention may be arranged on an inner surface of the enclosure.

Furthermore, the protective layer or the light emitting arrangement according to embodiments of the invention may also be employed in luminaires comprising solid-state light sources, such as LED-based luminaires. Such a luminaire may have any suitable design and be fitted for being suspended from above (e.g. a ceiling, as in Figure 7), mounted on a wall or standing on a surface. Figure 7 shows a perspective view of a suspended luminaire 700, comprising a frame 701 and a light exit window 702. Arrows indicate the direction of light emission from the luminaire 700. A light emitting arrangement (not shown) including at least one light source, typically a plurality of LEDs, may be mounted within the frame, behind the light exit window to emit light in the direction of the light exit window 702. A protective layer or the layer assembly according to embodiments of the invention may be arranged at any suitable position between the light source(s) and the light exit window 702. It is contemplated that the protective layer or layer assembly according to embodiments of the invention may be arranged on an inner surface of the light exit window or form part of the light exit window.

The light emitting arrangement according to embodiments of the invention may also be employed in LED modules intended for any type of lighting application.

For example, any embodiment of a light emitting arrangement according to the invention may comprise a plurality of solid state light sources. Furthermore, a protective layer comprising the organometallic getter need not be the only protective layer of e.g. a light emitting arrangement. Indeed, the protective layer may be contained in a hermetically or pseudo-hermetically sealed compartment, typically together with other components such as a light source, a reflector, a wavelength converting material, etc. Sealing may be achieved by one or more additional sealing material(s) or sealing layer(s) known in the art.

Additionally, variations to the disclosed embodiments can be understood and effected by the skilled person in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage.

## Claims

1. A protective composition for use in an optoelectronic device, said protective composition comprising an organometallic getter capable of reacting with sulfur and/or selenium to form crystals containing a metal and sulfur and/or selenium according to the following equation:
H₂Z + MX → MZ + XH₂
wherein H₂Z represents sulfide gas or selenide gas, M represents a metal moiety of the organometallic getter selected from the group consisting of copper, iron, cadmium, lead and zinc, X represents an organic moiety of the organometallic getter, and MZ represents the crystals.

2. A protective composition according to claim 1, wherein said organometallic getter comprises zinc.

3. A protective composition according to claim 1, wherein said organometallic getter comprises an organic anion.

4. A protective composition according to claim 1, further comprising a matrix comprising a polymer.

5. A protective composition according to claim 4, wherein said organometallic getter is chemically attached to the polymer.

6. A protective composition according to claim 1, further comprising a wavelength converting material.

7. A protective layer comprising a protective composition according to claim 1.

8. A layer assembly comprising a protective layer according to claim 7 and a wavelength converting layer comprising a wavelength converting material.

9. A protective composition according to claim 6, or a layer assembly according to claim 7, wherein said wavelength converting material comprises sulfur and/or selenium.

10. An optical component comprising a protective composition according to claim 1.

11. A light emitting arrangement comprising at least one solid state light source and a protective composition according to claim 1.

12. A light emitting arrangement according to claim 11, further comprising a silver layer, wherein said silver layer is at least partly covered by said protective composition.

13. A light emitting arrangement according to claim 11, further comprising a wavelength converting material arranged to receive light emitted by said solid state light source, wherein said wavelength converting material is at least partly covered by said protective composition.

14. Use of an organometallic getter for capturing corrosive or toxic gas released from a wavelength converting material.

## Patentansprüche

1. Schutzzusammensetzung zur Verwendung in einer optoelektronischen Vorrichtung, wobei die Schutzzusammensetzung einen metallorganischen Getter umfasst, der imstande ist, mit Schwefel und/oder Selen zu reagieren, um Kristalle zu bilden, die ein Metall und Schwefel und/oder Selen gemäß der folgenden Gleichung enthalten:
H₂Z + MX → MZ + XH₂
wobei H₂Z Sulfidgas oder Selenidgas darstellt, M einen Metallrest des metallorganischen Getters darstellt, ausgewählt aus der Gruppe bestehend aus Kupfer, Eisen, Kadmium, Blei und Zink, X einen organischen Rest des metallorganischen Getters darstellt, und MZ die Kristalle darstellt.

2. Schutzzusammensetzung nach Anspruch 1, wobei der metallorganische Getter Zink umfasst.

3. Schutzzusammensetzung nach Anspruch 1, wobei der metallorganische Getter ein organisches Anion umfasst.

4. Schutzzusammensetzung nach Anspruch 1, ferner umfassend eine Matrix, die ein Polymer umfasst.

5. Schutzzusammensetzung nach Anspruch 4, wobei der metallorganische Getter mit dem Polymer chemisch verbunden ist.

6. Schutzzusammensetzung nach Anspruch 1, ferner umfassend ein Wellenlängen-konvertierendes Material.

7. Schutzschicht, umfassend eine Schutzzusammensetzung nach Anspruch 1.

8. Schichtanordnung, umfassend eine Schutzschicht nach Anspruch 7 und eine Wellenlängen-konvertierende Schicht, die ein Wellenlängen-konvertierendes Material umfasst.

9. Schutzzusammensetzung nach Anspruch 6 oder Schichtanordnung nach Anspruch 7, wobei das Wellenlängen-konvertierende Material Schwefel und/oder Selen umfasst.

10. Optische Komponente, umfassend eine Schutzzusammensetzung nach Anspruch 1.

11. Licht-emittierende Anordnung, umfassend mindestens eine Festkörper-Lichtquelle und eine Schutzzusammensetzung nach Anspruch 1.

12. Licht-emittierende Anordnung nach Anspruch 11, ferner umfassend eine Silberschicht, wobei die Silberschicht mindestens teilweise von der Schutzzusammensetzung bedeckt ist.

13. Licht-emittierende Anordnung nach Anspruch 11, ferner umfassend ein Wellenlängen-konvertierendes Material, das angeordnet ist, um Licht zu empfangen, das von der Festkörper-Lichtquelle ausgestrahlt wird, wobei das Wellenlängen-konvertierende Material mindestens teilweise von der Schutzzusammensetzung bedeckt ist.

14. Verwendung eines metallorganischen Getters zum Auffangen von korrosivem oder giftigem Gas, das von einem Wellenlängen-konvertierenden Material freigesetzt wird.

## Revendications

1. Composition protectrice à utiliser dans un dispositif optoélectronique, ladite composition protectrice comprenant un getter organométallique capable de réagir avec du soufre et/ou du sélénium afin de former des cristaux contenant un métal et du soufre et/ou du sélénium selon l'équation suivante :
H₂Z + MX → MZ + XH₂
dans laquelle H₂Z représente du sulfure gazeux ou du séléniure gazeux, M représente un fragment de métal du getter organométallique sélectionné dans le groupe constitué de cuivre, de fer, de cadmium, de plomb et de zinc, X représente un fragment organique du getter organométallique, et MZ représente les cristaux.

2. Composition protectrice selon la revendication 1, dans laquelle ledit getter organométallique comprend du zinc.

3. Composition protectrice selon la revendication 1, dans laquelle ledit getter organométallique comprend un anion organique.

4. Composition protectrice selon la revendication 1, comprenant en outre une matrice comprenant un polymère.

5. Composition protectrice selon la revendication 4, dans laquelle ledit getter organométallique est chimiquement attaché au polymère.

6. Composition protectrice selon la revendication 1, comprenant en outre un matériau de conversion de longueur d'onde.

7. Couche protectrice comprenant une composition protectrice selon la revendication 1.

8. Ensemble de couches comprenant une couche protectrice selon la revendication 7 et une couche de conversion de longueur d'onde comprenant un matériau de conversion de longueur d'onde.

9. Composition protectrice selon la revendication 6, ou ensemble de couches selon la revendication 7, dans lequel ledit matériau de conversion de longueur d'onde comprend du soufre et/ou du sélénium.

10. Composant optique comprenant une composition protectrice selon la revendication 1.

11. Agencement d'émission de lumière comprenant au moins une source de lumière à semi-conducteurs et une composition protectrice selon la revendication 1.

12. Agencement d'émission de lumière selon la revendication 11, comprenant en outre une couche d'argent, dans lequel ladite couche d'argent est au moins partiellement recouverte de ladite composition protectrice.

13. Agencement d'émission de lumière selon la revendication 11, comprenant en outre un matériau de conversion de longueur d'onde agencé pour recevoir la lumière émise par ladite source de lumière à semi-conducteurs, dans lequel ledit matériau de conversion de longueur d'onde est au moins partiellement recouvert de ladite composition protectrice.

14. Utilisation d'un getter organométallique pour capturer le gaz corrosif ou toxique dégagé d'un matériau de conversion de longueur d'onde.
